Europäisches Patentamt
European Patent Office
Office européen des brevets

(11) Publication number: 0 273 696 A2

(12) # EUROPEAN PATENT APPLICATION

(21) Application number: 87311336.9

(22) Date of filing: 22.12.87

(51) Int. Cl.⁴: **C 07 K 5/02**
C 07 D 233/64, A 61 K 37/64,
A 61 K 31/415

(30) Priority: 02.01.87 US 21

(43) Date of publication of application:
06.07.88 Bulletin 88/27

(84) Designated Contracting States:
AT BE CH DE ES FR GB IT LI LU NL SE

(71) Applicant: MERCK & CO. INC.
126, East Lincoln Avenue P.O. Box 2000
Rahway New Jersey 07065-0900 (US)

(72) Inventor: Greenlee, William J.
115 Herrick Avenue
Teaneck New Jersey 07666 (US)

Patchett, Arthur A.
1090 Minisink Way
Westfield New Jersey 07090 (US)

Parsons, William H.
2171 Oliver Street
Rahway New Jersey 07065 (US)

Chakravarty, Prasun K.
26 Wintergreen Avenue
Edison New Jersey 08820 (US)

(74) Representative: Hesketh, Alan, Dr.
European Patent Department Merck & Co., Inc. Terlings
Park Eastwick Road
Harlow Essex, CM20 2QR (GB)

(54) **Renin inhibitors containing histidine replacements.**

(57) Peptides of the formula:

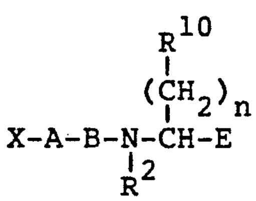

,

which comprises novel elements replacing the His(9) sequence in renin-inhibitory peptides based on substrate analogy, which have improved bioavailability and pharmacodynamic properties; compositions containing these renin-inhibitory peptides, optionally with other antihypertensive agents; and methods of treating hypertension or congestive heart failure or of establishing renin as a causative factor in these problems employing the novel peptides.

EP 0 273 696 A2

**Description**

## RENIN INHIBITORS CONTAINING HISTIDINE REPLACEMENTS

The present invention is concerned with novel peptides which inhibit the proteolytic enzyme, renin, with pharmaceutical compositions containing the novel peptides of the present invention as active ingredients, and with methods of treating hypertension and congestive heart failure and methods of diagnosis which utilize the novel peptides of the present invention.

### BACKGROUND OF THE INVENTION

Renin is an endopeptidase secreted by the juxtaglomerular cells of the kidney, which cleaves its plasma substrate, angiotensinogen, specifically at the 10, 11 peptide bond, i.e., between Leu 10 and Leu 11 in the equine substrate, as described by Skeggs et al, J. Exper. Med. 1957, 106, 439, or between the Leu 10 and Val 11 in the human renin substrate, as elucidated by Tewksbury et al., Circulation 59, 60, Supp. II: 132, Oct. 1979. Renin cleaves angiotensinogen to split off the decapeptide, angiotensin I, which is converted by angiotensin-converting enzyme to the potent pressor substance angiotensin II. Thus, the renin-angiotensin system plays an important role in normal cardiovascular homeostasis and in some forms of hypertension.

Inhibitors of angiotensin I converting enzyme have proven useful in the modulation of the renin-angiotensin system and consequently, specific inhibitors of the limiting enzymatic step that ultimately regulates angiotensin II production, the action of renin on its substrate, have also been sought as effective investigative tools and as therapeutic agents in the treatment of hypertension and congestive heart failure.

Renin antibody, pepstatin, phospholipids, and substrate analogs, including tetrapeptides and octa- to tridecapeptides, with inhibition constants ($K_i$) in the $10^{-3}$ to $10^{-6}$M region, have been studied.

Umezawa et al., in J. Antibiot. (Tokyo) 23: 259-262, 1970, reported the isolation of a peptide, pepstatin, from actinomyces that was an inhibitor of aspartyl proteases such as pepsin, cathepsin D, and renin. Gross et al., Science 175:656, 1972, reported that pepstatin reduces blood pressure in vivo after the injection of hog renin into nephrectomized rats, but pepstatin has not found very wide application as an experimental agent because of its limited solubility and its inhibition of a variety of other acid proteases in addition to renin.

Many efforts have been made to prepare a specific renin inhibitor based on pig renin substrate analogy, which as been shown to correlate well with and predict human renin inhibitor activity. The octapeptide amino acid sequence extending from histidine-6 through tyrosine-13

$$\left(\begin{matrix} 6 & 7 & 8 & 9 & 10 & 11 & 12 & 13 \\ \text{-His-} & \text{Pro-} & \text{Phe-} & \text{His-} & \text{Leu-} & \text{Leu-} & \text{Val-} & \text{Tyr-} \end{matrix}\right)$$

has been shown to have kinetic parameters essentially the same as those of the full tetradecapeptide renin substrate.

Kokubu et al., Biochem. Pharmacol., 22, 3217-3223, 1973, synthesized a number of analogs of the tetrapeptide found between residues 10 to 13, but while inhibition could be shown, inhibitory constants were only of the order of $10^{-3}$M. Analogs of a larger segment of renin substrate were synthesized, Burton et al., Biochemistry 14: 3892-3898, 1975, and Poulsen et al., Biochemistry 12: 3877-3882, 1973, but a lack of solubility and weak binding (large inhibitory constant) have proven to be major obstacles to obtaining effective renin inhibitors.

Modifications to increase solubility soon established that the inhibitory properties of the peptides are markedly dependent on the hydrophobicity of various amino acid residues, and that increasing solubility by replacing lipophilic amino acids with hydrophilic isosteric residues becomes counter-productive. Other approaches to increasing solubility have had limited success.

Modifications designed to increase binding to renin have also been made, but here too, with mixed results.

Powers et al., in Acid Proteases, Structure, Function and Biology, Plenum Press, 1977, 141-157, have suggested that in pepstatin, statine occupies the space of the two amino acids on either side of the cleavage site of a pepsin substrate, and Tang et al., in Trends in Biochem. Sci., 1:205-208 (1976) and J. Biol. Chem., 251:7088-94, 1976, have proposed that the statine residue of pepstatin resembles the transition state for pepsin hydrolysis of peptide bonds. Inhibitors of renin which contain the amino acid statine have been disclosed in the following: Veber et al, U.S. Patent 4,384,994; European Published Application 77 029; Evans et al, U.S. Patent 4,397,786; Veber et al, EP-A 77 028; Boger et al, Nature, 1983, 303, 81-84; U.S. Patent 4,470,971; EP-A 114 993 and 157 409; U.S. Patent 4,485,099; Matsueda et al, EP-A 128 762, 152 225; Morisawa et al., EP-A 186 977; Riniker et al, EP-A 111 266; Bindra et al, EP-A 155 809; Stein et al, Fed. Proc. 1986, 45, 869; and German Patent Application DE 3438-545-A.

Renin inhibitors containing other peptide bond isosteres, including a reduced carbonyl peptide bond isostere are disclosed by M. Szelke et al, in work described in published European Patent Application 45 665, 104 041, U.S. Patent 4,424,207, and in PCT Int. Appl. WO 84 03,004; Nature, 299, 555 (1982); Hypertension, 4, Supp. 2, 59, 1981; British Patent 1,587,809; and in Peptides, Structure and Function.: Proceedings of the Eighth American Peptide Symposium, ed. V. J. Hruby and D. H. Rich, p. 579, Pierce Chemical Co., Rockford,

IL., 1983, where substitution at the Leu-Leu site of renin cleavage by isosteric substitution, resulted in compounds with excellent potency. Other peptide bond isosteres have been disclosed in Buhlmayer et al in EP-A 144 290 and 184 550; Hester et al, EP-A 173 481; Raddatz, EP-A 161 588; Dann et al, Biochem. Biophys. Res. Commun. 1986, 134, 71-77; Fuhrer et al, EP-A 143 746; Kamijo et al, EP-A 181 110; Thaisrivongs et al, J. Med. Chem., 1985, 28, 1553-1555; and Ryono et al., EP-A 181 071.

Renin inhibitors with non-peptide C-termini are disclosed in European Published Applications 172 346 and 172 347.

Peptide aldehyde renin inhibitors are disclosed by Kokubu et al, Hypertension, 1985, 7, Suppl. I, p. 8-10; Matsueda et al, Chemistry Letters, 1985, 1041-1044; European Published Applications 128 762 and 152 225. Peptide glycol inhibitors of renin are reported by Hanson et al in Biochem. Biophys. Res. Commun. 1985, 132, 155-161.

The renin inhibitors cited above in large part comprise peptide-based inhibitors in which a sequence of the type: ...A-B-D-E-F-G-J-K-L... , where G is a peptide bond mimic and A,B,D,E,F,J,K, and L may individually be absent or may represent naturally-occuring or modified amino acids. Typical sequences of this type include:

```
       7   8   9   10  11  12
...BOC-Pro-Phe-His-Sta-Leu-Phe... ,

           8   9   10  11
or  ...BOC-Phe-His-Sta-Leu... ,
```

where the N-terminus typically comprises an amino acid protecting group such as BOC or CBZ, and the N-terminal amino acids are Pro-Phe-His or Phe-His.

Replacements for the His(9) portion of these sequences have been described among these references, wherein amino acids (e.g., Lys, Leu or $NH_2$-CH ($CH_2$Het)$CO_2$H, wherein "Het" is a mono- or bicyclic heterocycle) have been substituted into substrate-based sequences as replacements for His. These 9-substituted sequences include peptides in which various peptide bonds have been N-methylated or reduced in order to stabilize the resulting inhibitor against enzymatic degradation, but otherwise, no advantage in renin-inhibitory potency or in pharmacological properties has been demonstrated or suggested by making such substitutions for the His element.

## DESCRIPTION OF THE INVENTION

The present invention discloses renin inhibitors demonstrating improved bioavailability and improved pharmacodynamic properties, in which a novel element, replacing the histidine (9) residue, is incorporated into a peptide inhibitor containing statine or another peptide bond isostere.

In particular, the present Invention is directed to renin-inhibitory peptides of the formula:

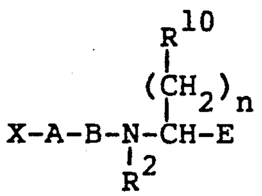

(I),

wherein

X is hydrogen, $C_1$-$C_5$-alkyl, $R^1OCH_2CO$, $R^1CH_2OCO$, $R^1OCO$, $R^1(CH_2)_nCO$, $(R^2)_2N(CH_2)_nCO$, $R^1SO_2(CH_2)_mCO$, $R^1SO_2(CH_2)_mOCO$, $R^1OCHCO$, $(R^1)_2CHCO$, $R^1NHCHCO$, or $R^1SCHCO$, where

$R^1$ is $C_1$-$C_6$-alkyl; $C_3$-$C_7$-cycloalkyl; aryl, wherein aryl is unsubstituted or mono-, di- or trisubstituted phenyl or naphthyl, where the substituent(s) is/are independently selected from the group consisting of amino mono- or di-$C_1$-$C_4$-alkylamino, amino-$C_1$-$C_4$-alkyl, hydroxy-$C_1$-$C_4$-alkyl, di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$-alkyl, $NR^5R^6$, and $N(R^5)_3^{\oplus} A^{\ominus}$, wherein $R^5$ and $R^6$ are independently hydrogen, unsubstituted or monosubstituted $C_1$-$C_4$-alkyl, where the substituent is amino, $C_1$-$C_4$-alkylamino, di-$C_1$-$C_4$-alkylamino, hydroxyl, $C_1$-$C_4$-alkoxy or $N(C_1$-$C_4$-alkyl$)_3^{\oplus}A^{\ominus}$, and $A^{\ominus}$ is a counterion selected from the group consisting of single negatively-charged ions, such as chloride, bromide, hydroxide and acetate; Het, wherein Het is an unsubstituted or mono- or disubstituted 5- or 6-membered heterocyclic ring or benzofused 5- or 6- membered heterocyclic ring, where the one or two heteroatoms are independently selected from the group consisting of N, O, S, NO, SO, $SO_2$ or quarternized N, and the substituent(s) is/are independently selected from the group consisting of hydroxyl, thiol, $C_1$-$C_6$-alkyl, $CF_3$, $C_1$-$C_4$-alkoxy, halo, aryl, aryl-$C_1$-$C_4$-alkyl, amino, mono- or di-$C_1$-$C_4$-alkylamino, amino-$C_1$-$C_4$-alkylamino, amino-$C_1$-$C_4$-alkyl, hydroxy-$C_1$-$C_4$-alkyl, di-$C_1$-$C_4$-alkyl-amino-$C_1$-$C_4$-alkyl, guanidyl, guanidyl-$C_1$-$C_4$-alkyl,

CHO, $CO_2H$, $CO_2$-$C_1$-$C_4$-alkyl, $NR^5R^6$, and $N(R^5)_3^{\oplus}A^{\ominus}$, wherein $R^5R^6$ and $A^{\ominus}$ are as defined above, or 5-oxo-2-pyrrolidinyl;

$R^2$ is hydrogen or $C_1$-$C_5$-alkyl;

m is 1 to 5;

n is 0 to 5;

A is absent;

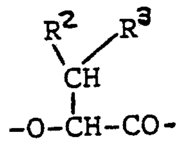

where

$R^3$ is hydrogen; $C_1$-$C_5$-alkyl; $C_3$-$C_7$-cycloalkyl; 1- or 2-adamantyl; $(CH_2)_p$-aryl, wherein aryl is as defined above and p is 1 to 3; or $(CH_2)_p$-Het, wherein Het and p are as defined above; and

$R^2$ is as defined above;

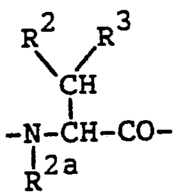

where

$R^{2a}$ is hydrogen or $C_1$-$C_5$-alkyl; and

$R^2$ and $R^3$ are as defined above;

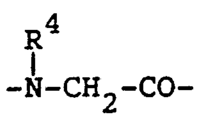

where

$R^4$ is hydrogen, $C_1$-$C_5$-alkyl or aryl-$C_1$-$C_5$-alkyl, wherein aryl is as defined above;

B is:

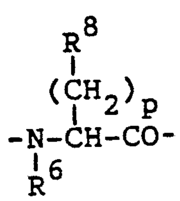 ,

where

$R^6$ is aryl-substituted $C_1$-$C_5$-alkyl, wherein aryl is as defined above, $C_5$-$C_7$-cycloalkyl, or Het, as defined above;

$R^8$ is unsubstituted or monosubstituted $C_1$-$C_4$-alkyl, wherein the substituent is carboxyl, amino, hydroxyl, guanidino, $C_1$-$C_4$-alkoxy-carbonyl, Het, as defined above, or aryl, as defined above; and

p is as defined above;

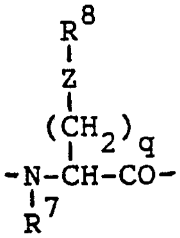

where

$R^7$ is hydrogen, unsubstituted or aryl-substituted $C_1$-$C_5$-alkyl, wherein aryl is as defined above, $C_5$-$C_7$-cycloalkyl, or Het, as defined above;

Z is O, S, $NR^2$, $NR^2$-CO,CO-$NR^2$, SO, $SO_2$, wherein $R^2$ is as defined above;

q is 0 to 3; and

$R^8$ is as defined above;

except that -$(CH_2)_q$,-Z-$R^8$ cannot be -$CH_2SCH_3$;

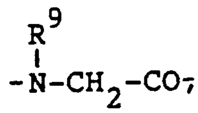

where

$R^9$ is $C_1$-$C_4$-alkyl, substituted by $R^8$ or Z-$R^8$, where $R^8$ and Z are as defined above;

except that $R^9$ cannot be hydrogen, $C_1$-$C_5$-alkyl or aryl-$C_1$-$C_5$-alkyl;

$R^{10}$ is -$CHR^{11}R^{11a}$;

where

$R^{11}$ and $R^{11a}$ are related such that when $R^{11}$ is hydrogen, $C_1$-$C_5$-alkyl, aryl, as defined above, Het, as defined above, unsubstituted or mono-, di or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy, and halo; $C_1$-$C_3$-alkoxy, or $C_1$-$C_3$-alkylthio, then $R^{11a}$ is hydrogen,

or when $R^{11}$ is hydroxy-$C_1$-$C_4$-alkyl, amino-$C_1$-$C_4$-alkyl, or $C_1$-$C_4$-alkyl, then $R^{11a}$ is hydrogen or $C_1$-$C_3$-alkyl;

E is - CH(W)-G,

where

W is OH, $NH_2$ or $OR^{12}$, wherein $R^{12}$ is $C_1$-$C_4$-alkanoyl or $C_1$-$C_6$-alkanoyloxy-$C_1$-$C_4$-alkyl; and

G is Q-CO-T-U-V,

wherein

Q is a single bond; CH-$R^{13}$, where $R^{13}$ is hydrogen; aryl, as defined above; $C_1$-$C_8$-alkyl; $C_3$-$C_7$-cycloalkyl; mono- or disubstituted $C_2$-$C_8$-alkyl, where the substi tuent(s) is/are independently selected from the group consisting of hydroxy; $CO_2H$; $CO_2$-$C_1$-$C_4$-alkyl; $CONR^5R^6$, wherein $R^5$ and R are as defined above; amino; mono- or di-$C_1$-$C_4$-alkylamino; and guanidyl, and the substitution occurs on the last 1 or 2 carbon atoms of the alkyl chain; or mono- or disubstituted aryl or $C_3$-$C_7$-cycloalkyl,where the substituent(s) is/are independently selected from the group consisting of $C_1$-$C_4$-alkyl, trifluoromethyl, hydroxy, $C_1$-$C_4$-alkoxy, and halo; or $CH_2$-CH-$R^{13a}$, where $R^{13a}$ is $CHR^{14}R^{14a}$, wherein $R^{14}$ is hydrogen; amino; hydroxyl; $C_1$-$C_5$-alkyl; $C_3$-$C_7$-cycloalkyl; aryl, as defined above; aryl-$C_1$-$C_4$-alkyl; Het, as defined above; Het-$C_1$-$C_4$-alkyl; amino-$C_1$-$C_4$-alkyl; or guanidyl-$C_1$-$C_4$-alkyl; and

$R^{14a}$ is hydrogen or $C_1$-$C_5$-alkyl; T and U are independently absent or

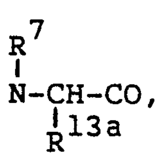

where $R^7$ and $R^{13a}$ are as defined above; and

V is O-$(CH_2)_uR^{16}$, where $R^{16}$ is hydrogen; aryl, as defined above; Het, as defined above; or unsubstituted or mono- or disubstituted $C_2$-$C_4$-alkyl, $C_3$-$C_7$-cycloalkyl or $C_2$-$C_7$-cycloalkyl-$C_1$-$C_4$-alkyl, wherein the

substituent(s) is/are independently selected from the group consisting of amino, hydroxyl, $C_1$-$C_4$-alkoxy, thio, halo, mono- or di-$C_1$-$C_4$-alkylamino, $CO_2H$, or $CONR^5R^6$, where $R^5$ and $R^6$ are as defined above; and u is 0 to 5;

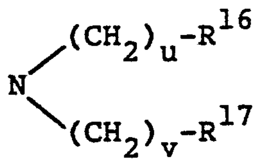 , where $R^{17}$ is hydrogen;

where $R^{17}$ is hydrogen; aryl, as defined above; Het, as defined above; unsubstituted or mono- or disubstituted $C_2$-$C_4$-alkyl, $C_3$-$C_7$-cycloalkyl or $C_3$-$C_7$-cycloalkyl-$C_1$-$C_4$-alkyl, wherein the substituent(s) is/are independently selected from the group consisting of amino; hydroxyl; $C_1$-$C_4$-alkoxy; thio; halo; mono- or di-$C_1$-$C_4$-alkylamino; $CO_2H$; or $CONR^5R^6$, where $R^5$ and $R^6$ are as defined above;
v is 0 to 5; and
$R^{16}$ and u are as defined above; or $R^{16}$ and $R^{17}$ are joined to form an unsubstituted or mono- or disubstituted 5- to 7-membered heterocyclic ring or benzofused 5- to 7-membered heterocyclic ring, which is either saturated or unsaturated, wherein the one to three heteroatoms is/are independently selected from the group consisting of nitrogen, oxygen and sulfur, and the substituent(s) is/are independently selected from the group consisting of $C_1$-$C_6$-alkyl; hydroxyl; trifluoromethyl; $C_1$-$C_4$-alkoxy; halo; aryl, as defined above; aryl-$C_1$-$C_4$-alkyl; amino; thio; and mono- or di-$C_1$-$C_4$-alkylamino;

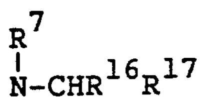,

where $R^7$, $R^{16}$ and $R^{17}$ are as defined above, $R^{16}$ or $R^{17}$ are joined as defined above; or $O$-$CHR^{16}R^{17}$, where $R^{16}$ and $R^{17}$ are as defined above, or are joined as defined above; or

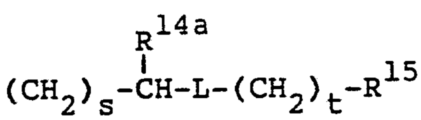,

wherein
$R^{15}$ is hydrogen; unsubstituted or mono-substituted $C_1$-$C_6$-alkyl, where the substituent is amino or hydroxyl; aryl-$C_1$-$C_4$-alkyl, where aryl is as defined above; $C_5$-$C_7$-cycloalkyl-$C_1$-$C_4$-alkyl; $C_1$-$C_5$-alkenyl; or Het, as defined above;
L is a single bond, S, SO, $SO_2$, $NR^7$, or $NR^7$-CO, where $R^7$ is as defined above;
s is 0 to 2;
t is 0 to 2; and
$R^{14a}$ is as defined above;

$CH_2$-$NR^{12a}$-M,
where
$R^{12a}$ is hydrogen, $C_1$-$C_5$-alkyl, $SO_2$-phenyl, formyl, or $CO_2$-$C_1$-$C_4$-alkyl; and

M is 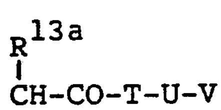,

wherein
$R^{13a}$, T, U and V are as defined above;
or
CO-$CF_2$-CO-T-U-V
where
T, U and V are as defined above;
and pharmaceutically-acceptable salts thereof.

As used herein, "alkyl" is intended to include both branched- and straight-chain saturated hydrocarbon groups having the specified number of carbon atoms; "alkoxy" represents an alkyl group of indicated number

of carbon atoms attached through an oxygen bridge; and "$C_3$-$C_7$-cycloalkyl" is intended to include cycloalkyl groups, such as cyclopropyl, cyclobutyl, cyclopentyl, cyclohexyl and cycloheptyl. "Alkanoyl" is intended to include those alkylcarbonyl groups of specified number of carbon atoms, which are exemplified by formyl, acetyl, propanoyl and butanoyl; and "arylalkyl" represents aryl groups as herein defined which are attached through a straight- or branched-chain alkyl group of specified number of carbon atoms, such as, for example, benzyl, phenethyl, 3,3-diphenylpropyl, 3-indolylmethyl, and the like. "Halo", as used herein, means fluoro, chloro, bromo and iodo, and "counterion" is used to represent a small, single negatively-charged specie, such as chloride, bromide, hydroxide, acetate, perchlorate, and the like.

As used herein, "aryl" is intended to mean phenyl or naphthyl, which is optionally-substituted by from one- to three- members independently selected from the group consisting of amino, mono- or di-$C_1$-$C_4$-alkylamino, amino-$C_1$-$C_4$-alkyl, hydroxy-$C_1$-$C_4$-alkyl, $C_1$-$C_4$-dialkyamino-$C_1$-$C_4$-alkyl, guanidyl, guanidyl $C_1$-$C_4$-alkyl, hydroxyl, $C_1$-$C_4$- alkoxy, $CF_3$, halo, CHO, $CO_2H$, $CO_2H$, $CO_2R^7$, $NR^5R^6$, wherein $R^7$, $R^5$ and $R^6$ are as defined above, or $N(R^5)_3^{\oplus}A^{\ominus}$, wherein $R^5$ is as defined above and $A^{\ominus}$ is counterion, as defined herein.

The term "Het", as used herein, represents a 5- or 6-membered heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quarternized, and including any bicyclic group in which any of the above-defined heterocyclic rings is fused to a benzene ring. In the case of a heterocyclic ring containing one or more nitrogen atoms, the point of attachment may be at one of the nitrogen atoms, or at any carbon atom. Examples of such heterocyclic elements include piperidyl, piperazinyl, 2-oxopiperazinyl, pyrryl, 4-piperidonyl, pyrrolidinyl, pyrazolyl, pyrazolinyl, imidazolyl, imidazolinyl, pyridyl, pyrazinyl, pyrimidinyl, pyridazinyl, oxaolyl, oxazolidinyl, isoxazolyl, morpholinyl, thiazolyl, thiazolidinyl, indolyl, quinolinyl, furyl, thienyl, benzothienyl, morpholinyl, thiamorpholinyl, thiamorpholinyl sulfoxide, thiamorpholinyl sulfone. The heterocyclic moiety is further optionally-substituted by from one- to three- members independently selected from the group consisting of hydroxyl, thiol, $C_1$-$C_6$-alkyl, $CF_3$, $C_1$-$C_4$-alkoxy, halo, aryl, aryl-$C_1$-$C_4$-alkyl, amino, mono- or di-$C_1$-4-alkylamino, amino-$C_1$-$C_4$-alkylamino, amino-$C_1$-$C_4$-alkyl, hydroxy-$C_1$-$C_4$-alkyl, $C_1$-$C_4$-dialkylamino-$C_1$-$C_4$-alkyl, guanidyl, guanidyl $C_1$-$C_4$-alkyl, hydroxyl, CHO, $CO_2H$, $CO_2R^7$, $NR^5R^6$, wherein $R^7$, $R^5$ and $R^6$ are as defined above, or $N(R^5)_3^{\oplus}$ $A^{\ominus}$, wherein $R^5$ is as defined above and $A^{\ominus}$ is counterion, as defined herein.

The following additional abbreviations have also been used herein:

| Abbreviated Designation | Amino Acid/Residue |
|---|---|
| ACHPA | (3S,4S)-4-amino-5-cyclohexyl-3-hydroxypentanoic acid |
| Cys | cysteine |
| His | D or L-histidine |
| Leu | L-leucine |
| Lys | L-lysine |
| Phe | L-phenylalanine |
| Pro | proline |
| Sta | statine |
| Trp | L-tryptophan |
| Tyr | L-tyrosine |
| | **Protecting Group** |
| BOC | *t*-butyloxycarbonyl |
| BOM | benzyloxymethyl |
| CBZ | benzyloxycarbonyl(carbobenzoxy) |
| DNP | 2,4-dinitrophenyl |
| | **Activating Group** |
| HBT(HOBt) | 1-hydroxybenzotriazole hydrate |
| | **Condensing Agent** |
| DCCI (DCC) | dicyclohexylcarbodiimide |
| DPPA | diphenylphosphorylazide |

| | Reagent |
|---|---|
| AMP | 4-aminomethylpyridine |
| $(BOC)_2O$ | di-t-butyl dicarbonate |
| DEAD | diethyl azodicarboxylate |
| TFA | trifluoroacetic acid |

| | Solvent |
|---|---|
| AcOH (HOAc) | acetic acid |
| DMF | dimethylformamide |
| EtOAc (E) | ethyl acetate |
| $Et_2O$ | ether |

The A and B components in the renin inhibitors of the present invention have asymmetric centers and occur as racemates, racemic mixtures and as individual diastereomers, with all isomeric forms being included in the present invention. In general, the preferred chiral forms of the A and B components are those which correspond to the naturally-occurring L-amino acids. The chiral forms of the T and U components are those which correspond to either the naturally-occurring L-or D amino acids.

Preferred renin inhibitors according to the instant invention include those wherein

E is $\overset{W}{|}$ CH-G, (W attached to CH)

where W is OH and G is Q-CO-T-U-V, wherein Q is $CH_2$ or

$$\begin{matrix} & R^{13a} \\ & | \\ CH- & CH, \end{matrix}$$ where

where $R^{13a}$ is isopropyl or isobutyl, and either T or U is

$$\begin{matrix} NH_2 \\ | \\ (CH_2)_3 \\ | \\ NH-CH-CO \end{matrix} \quad \text{or} \quad \begin{matrix} NH_2 \\ | \\ (CH_2)_4 \\ | \\ NH-CH-CO. \end{matrix}$$

Particularly preferred renin inhibitors according to the instant invention then include:
BOC-(4-methoxy)Phe-[S-(2-amino-1,3,4-thiadiazol-5-yl)]Cys-ACHPA-NH-(2(S)-methylbutyl);
BOC-Phe-[S-(1,2,4-triazol-3-yl)]Cys-AmACHPA-NH-(2(S)-methylbutyl);
BOC-Phe-[S-2-aminothiazol-4-yl]Cys-ACHPA-AMP;
BOC-N-(3-phenylpropyl)His-(2-allyl)ACHPA-NH-(2(S)-methylbutyl);
BOC-Phe-[S-(1,2,4-triazol-3-yl]Cys-ACHPA-NH-([2(S)-methylbutyl);
BOC-Phe-[S-(4-pyridyl)]Cys-ACHPA-NH-[2(s)-methylbutyl];
BOC-Phe-Glu(qN,N-diethylamide]-ACHPA-NH-(2(S)-methylbutyl);
CBZ-Phe-[S-(3-amino-1,2,4-triazol-5-yl)]Cys-Cal-[CH(OH)$CH_2$]Val-Leu-AMP;
BOC-Phe-[S-(1-methyltetrazol-5-yl)]Cys-ACHPA-NH-(2(S)-methylbutyl);
BOC-Phe-[3-(N-benzylpyridin-4-yl)amino]Ala-ACHPA-Ile-AMP;
BOC-Phe-[S-(4-phenylthiazol-2-yl)]Cys-Cal[$CH_2$NH] Val-AMP;
BOC-[3-(1-Naphthyl)]Ala-S-(imidazol-2-yl)]Cys-ACHPA-NH-(2(S)-methylbutyl);
BOC-Phe-[S-(1,2,4-triazol-3-yl)]Cys-ACHPA-Lys-NH-i-butyl;
BOC-Phe-[S-(3-amino-1,2,4-triazol-5-yl)]Cys-ACHPA-Lys-NH-(2(S)-methylbutyl);
BOC-(p-O-methyl)Phe-[S-(1,2,4-triazol-3-yl)]Cys-Cal[CH (OH)$CH_2$]Val-Lys-NH-i-butyl;
BOC-Phe-[S-(1,2,4-triazol-3-yl)]Cys-Cal[CH(OH)$CH_2$]Val-Lys-O-t-butyl;
BOC-Phe-[S-(1,2,4-triazol-3-yl)]Cys-ACHPA-Ile-Lys-NH-i-butyl;
BOC-Phe-[S-(2-aminothiazol-3-yl)]Cys-ACHPA-Lys-NH-i-butyl;

BOC-Phe-[S-(1,2,4-triazol-3-yl)]Cys-ACHPA-Lys-Pro-$OCH_3$;
BOC-Phe-[S-(1,2,4-triazol-3-yl)]Cys-ACHPA-Lys-Phe-OH;
BOC-Phe-[S-(1,2,4-triazol-3-yl)]Cys-ACHPA-Lys-NH-i-butyl;
BOC-Phe-[S-(2-aminothiazol-3-yl)]Cys-ACHPA-Ile-Lys-;
BOC-Phe-[S-(2-aminothiazol-3-yl)]Cys-ACHPA-Lys-Lys-NH-i-butyl;
BOC-Phe-[S-(2-aminothiazol-3-yl)]Cys-ACHPA-Lys-NH-((1-hydroxymethyl)-5-aminopentyl);
BOC-Phe-Glu[q-N,N-diethylamide]-ACHPA-Lys-NH-i-butyl;
BOC-Phe-Lys[$N^{\varepsilon}$-acetyl]-ACHPA-Lys-NH-i-butyl;
BOC-Phe-Orn[$N^{\delta}$ -acetyl]-ACHPA-Lys-NH-i-butyl; and
BOC-Phe-Lys[$N^{\varepsilon}$-benzoyl]-ACHPA-Lys-NH-i-butyl.

Pharmaceutically-acceptable salts of the Formula I compounds include acid addition salts, such as acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxy-ethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate,tosylate, and undecanoate. The base salts of these compounds include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl and dibutyl; and diamyl sulfates or long chain halides such as decyl, lauryl, myristyl and stearyl chlorides, bromides and iodides or aralkyl halides like benzyl and phenethyl bromides and others. Conventional methods of preparing these water or oil-soluble or dispersible salts may be used.

There is further provided in the present invention a pharmaceutical composition for treating renin-associated hypertension and congestive heart failure, comprising a pharmaceutical carrier, optionally with an adjuvant, and a therapeuticaly-effective amount of a peptide of the formula I. The actual amount of active ingredient that may be combined with the carrier materials to produce a single dosage form will vary depending upon the host treated and the particular mode of administration.

Also, in accordance with the present invention there is still further provided a method of treating renin-associated hypertension and congestive heart failure, comprising administering to a patient in need of such treatment, a therapeutically-effective amount of a peptide of the formula I.

The renin-inhibitory peptides of the present invention may also be utilized as diagnostic methods for the purpose of establishing the significance of renin as a causative or contributory factor in hypertension or congestive heart failure in a particular patient. For this purpose the present peptides may be adminstered in a single dose of from 0.1 to 10 mg per kg of body weight.

Both in vivo and in vitro methods may be employed. In the in vivo method, a novel peptide of the present invention is administered to a patient, preferably by intravenous injection, although other routes of parenteral administration are also suitable, at a hypotensive dosage level and as a single dose, and there may result a transitory fall in blood pressure. This fall in blood pressure, if it occurs, then indicates supranormal plasma renin levels.

The novel inhibitors of the present invention may be viewed as consisting of an X-A-B fragment, composed of X-A and B elements, linked to an element R, giving X-A-B-R, where R is statine or other element, including the peptide bond mimics recited above. The element R may be the C-terminal element in the inhibitor or the inhibitor may consist of additional amino acids, R′, and R″ at the C-terminus. The preparation of the peptides X-A-B-R (or X-A-B-R-R′-R″) may then follow various procedures.

The peptide bond mimic, the statine or other peptide bond isostere which is element R, is prepared by the known methods as follows:

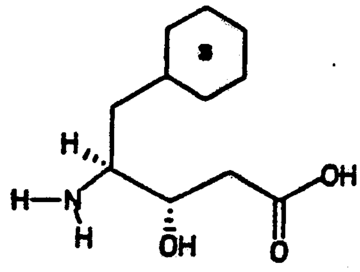

ACHPA

Boger et al. J. Med. Chem. 1985, 28, 1779-1790;

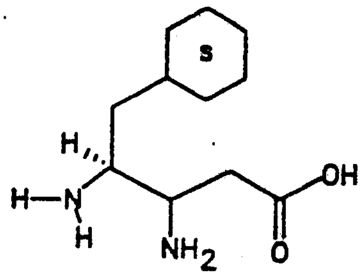

AmACHPA

Raddutz et al, Eur. Pat. Appl. 161 588;

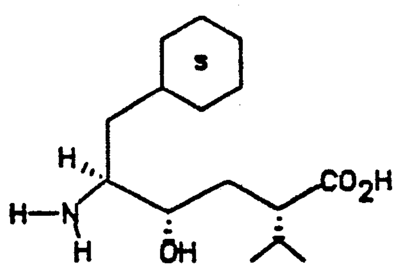

Cal[CH(OH)CH2]Val

Szelke et al, PCT Int. Appl. WO 84 03,044;

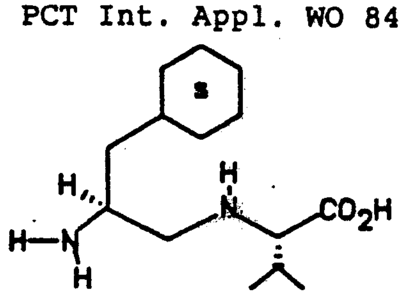

Cal[CH2NH]Val

European Pat. Appl. No. 173 481;

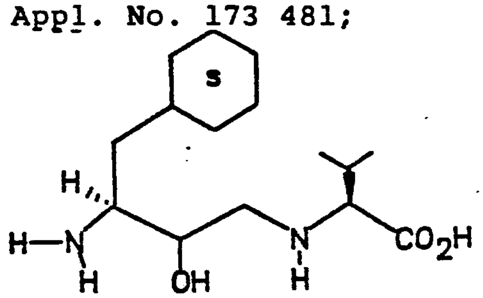

Cal[AA]Val

Dann et al, Biochem. Biophys. Res. Commun. 1986, 134, 71-77;

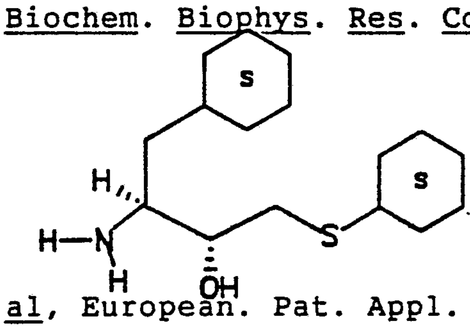

Plattner et al, European. Pat. Appl. No. 172 347;

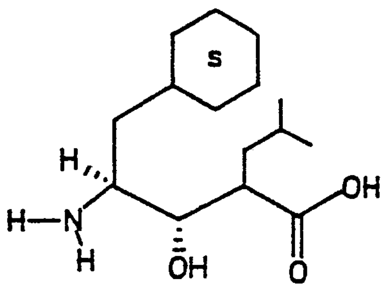

2-Substituted ACHPA

Boger et al, EP-A 0,157,409; Veber et al, Biochem. Soc. Trans., 1984, 12, 956-959; Stein et al, Fred Proc, 1986, 45, 869.

Using standard techniques of peptide synthesis, the R′ and R″ amino acids are coupled at the C-terminus of R. The R, R′, and R″ units may contain functionality which requires protection during synthesis of R and its coupling to R′ and R″, and protecting groups from among those well-known in peptide synthesis, are chosen so as to be compatible with these coupling steps, so as to also allow selective deprotection of the amino function in R, which is then to be coupled to the A-B fragment of the inhibitor.

These amino protecting groups are selectively removed from R, and then the synthesis proceeds by either coupling element B to R (with appropriate protection of B functionalities) and then coupling element X-A to B-R (or B-R-R′-R″), or by coupling element X-A to element B, followed by coupling of X-A-B to R (or R-R′-R″). Finally, protecting groups are removed from the inhibitor X-A-B-R-R′-R″ using standard techniques.

Throughout, protecting groups are chosen so as to be compatible with the coupling steps, yet easily removable afterwards. The BOC group is, in general, used to protect the amino groups involved in coupling steps, while the CBZ group is used to protect side-chain amino groups to be deprotected later, and carboxylic acids are protected as the methyl, ethyl, or benzyl esters thereof. The peptide coupling procedures used include those brought about by the use of dicyclohexylcarbodiimide [(DCC)/hydroxybenzotriazole(HOBt)] and of disuccinimido oxallate [(DSO)(Takeda et al, Tetrahedron Lett, 1983, 24, 4451-54)].

When the units A, B, R′ or R″ consist of N-alkyl (for example N-methyl) amino acids, procedures well-known in peptide synthesis are used to prepare these amino acids and to couple them. In general, the mixed

anhydride procedure (with pivaloyl chloride and N-methylmorpholine) is used, as illustrated by Wenger, Helv. Chem. Acta., 1984, 67, 502-525. N-methyl amino acids may be prepared, for example, by the method of O'Donnell, Tetrahedron Lett, 1984, 25, 3651, while N-alkyl amino acids generally may be prepared by the procedure of Freidinger, J. Org. Chem. 1983, 48, 77-81. N-substituted histidines may be prepared by the procedure of Stillwell, Bioorganic Chem., 1975, 4, 317-325.

The following schemes outline representative examples of the preparation of peptides of formula I, with similar peptides possessing alternative substituents being prepared by the routes outlined, with necessary modifications within the capabilities of those skilled in the art.

Preparation of the peptide of formula II may be carried out as follows:

SCHEME I

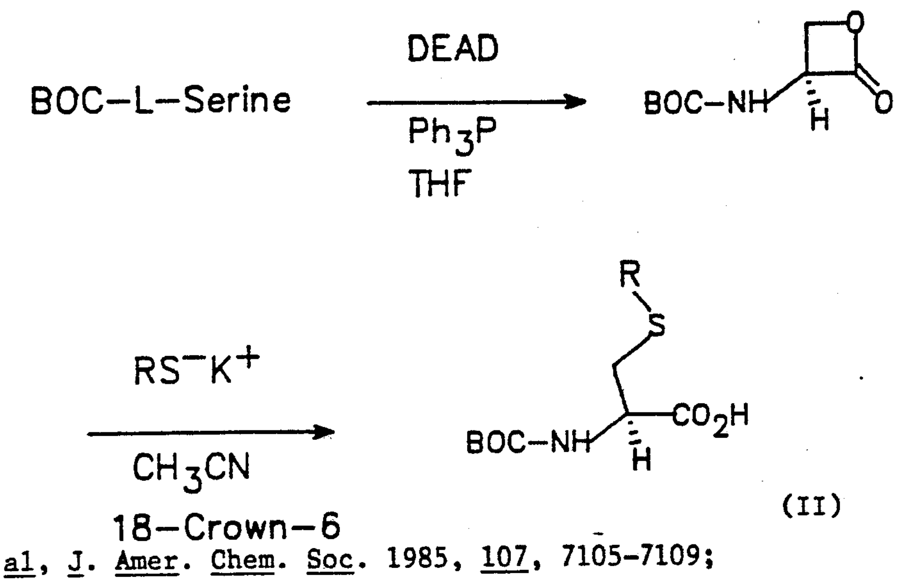


(II)

Arnold et al, J. Amer. Chem. Soc. 1985, 107, 7105-7109;

SCHEME II

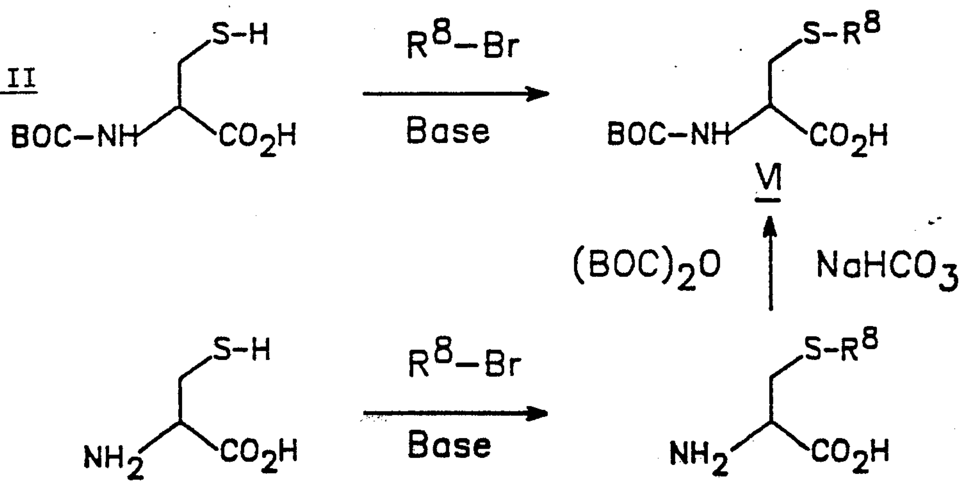


Frankel et al., J. Chem. Soc., 1960, 1390;

Akabori et al, Bull. Chem. Soc. Japan, 1964, 37, 433; and

Kornblum et al, J. Amer. Chem. Soc., 1974, 96, 590;

SCHEME III

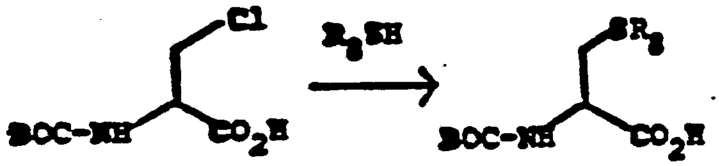


(II)

Melchor et al, Arch Biochem., 1947, 12, 301; and Wood et al, J. Biol. Chem., 1949, 179, 529.

Preparation of renin inhibitors of the present invention, which require a protected form of the B component, such as represented by the formulas III and IV, may be prepared as follows:

SCHEME IV

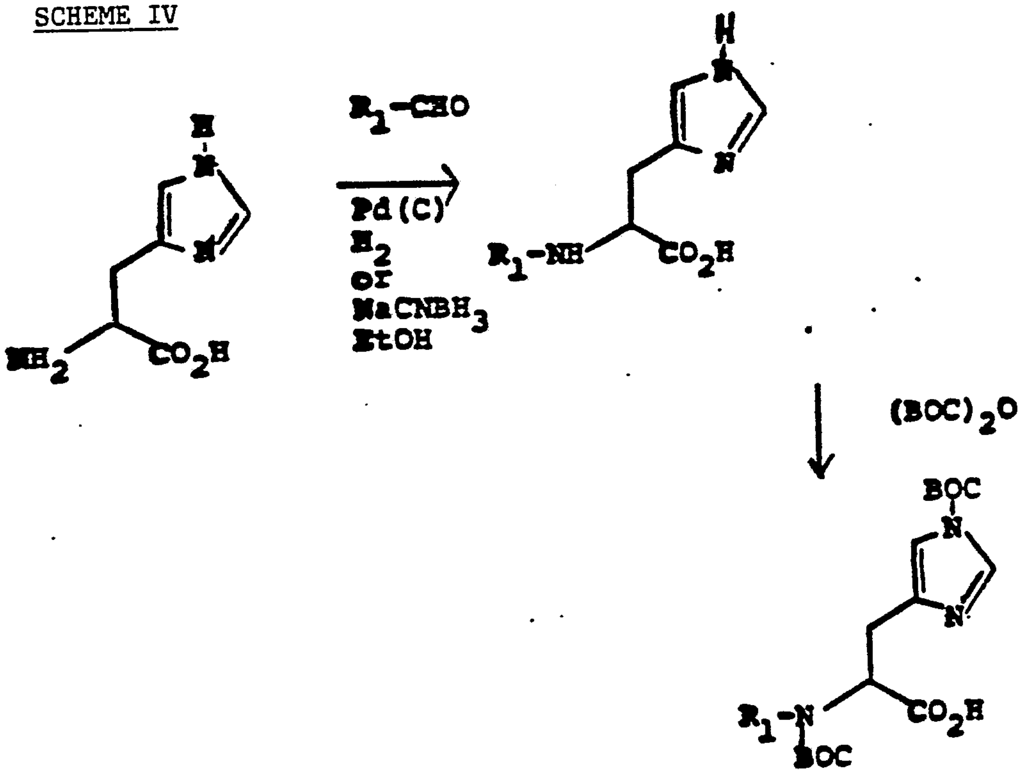


(III)

SCHEME V

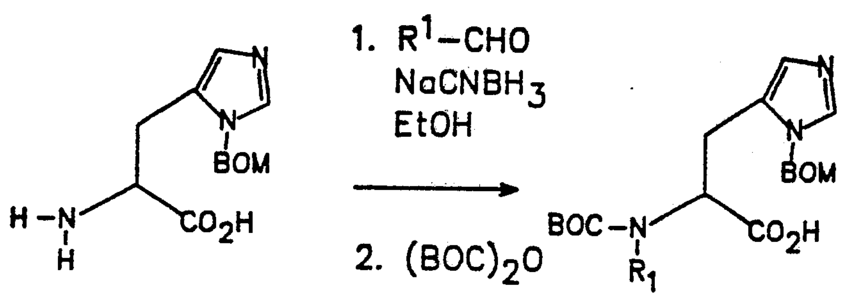


**Stillwell et al, Bioorganic Chem., 1975, 4, 317-325.**

The novel peptides of the present invention possess an excellent degree of activity in treating renin-associated hypertension and congestive heart failure in humans, as well as in other warm-blooded animals, such as mice, rats, horses, dogs, cats, etc., and they may be orally-active.

For these purposes, the compounds of the present invention may be administered orally, parenterally, by inhalation spray, or rectally in dosage unit formulations containing conventional non-toxic pharmaceutically-acceptable carriers, excipients, adjuvants and other vehicles. The term parenteral as used herein includes subcutaneous injections, intravenous, intramuscular, intrasternal injection or infusion techniques.

The pharmaceutical compositions may be in the form of a sterile injectable preparation, for example, as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example, as a solution in 1,3-butanediol.

Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The peptides of this invention may also be administered in the form of suppositories for rectal administration of the drug. These compositions can be prepared by mixing the drug with a suitable non-irritationg excipient, such as cocoa butter or polyethylene glycol, which is solid at ordinary temperatures by liquid at the rectal temperature and will therefore melt in the rectum to release the drug.

Dosage levels of the order of 0.1 to 4.0 grams per day parenterally are useful in the treatment of the above indicated conditions, with oral doses three-to-ten times higher. For example, renin-associated hypertension and hyperaldosteronism are effectively treated parenterally by the administration of from 1.0 to 50 milligram of the compound per kilogram of body weight per day. It will be understood, however, that the specific dose level for any particular patient will depend upon a variety of factors, including the activity of the specific compound employed, the age, body weight, general health, sex, diet, time of administration, route of administration, rate of excretion, drug combination and the severity of the particular disease undergoing therapy.

The present invention is also directed to combinations of the novel renin-inhibitory peptides of Formula I with one or more antihypertensive agents selected from the groups consisting of diuretics, and/or β-adrenergic blocking agents, CNS-acting antihypertensive agents, adrenergic neuron blocking agents, vasodilators, angiotensin I converting enzyme inhibitors, calcium channel blockers and other antihypertensive agents.

For example, the compounds of this invention can be given in combination with such compounds or salt or other derivative forms thereof as:

Diuretics:
acetazolamide; amiloride; benroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 or said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents:
dibenamine; phentolamine; phenoxybenzamine; prazosin; tolazoline;

B-Adrenergic Blocking Agents:
atenolol; metoprolol; nadolol; propranolol; timolol;
((±)-2-[3-(tert-butylamino)-2-hydroxypropoxyl]-2-furananilide)
(ancsarolol);(2-acetyl-7(2-hydroxy-3-isopropylaminoprop oxy)benzofuran HCl) (befunolo);
((±)-1-(isopropylamino)-3-(p-(2-cyclopropylmethoxyethyl)-phenoxy)-phenoxy)-2-propranol HCl) (betaxolol)
(1-[(3,4-dimethoxyphenethyl)amino]-3-(m-tolyloxy)-2-propanol HCl) (bevantolol);
(((±)-1-(4-((2-isopropoxyethoxy)methyl)phenoxy)-3-isopropylamino-2-propanol)fumarate) (bisoprolol);
(4-(2-hydroxy-3-[4-(phenoxymethyl)-piperidino]propoxy)-indole);
(carbazolyl-4-oxy-5,2-(2-methoxyphenoxy)-ethylamino-2-propanol);
(1-((1,1-dimethylethyl)amino)-3-((2-methyl-1H-indol-4-yl)oxy)-2-propanol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[(2-indol-3-yl-1,1-dimehylethyl)amino]propoxy]benzonitrile HCl) (bucindolol)
(a-[(tert.butylamino)methyl]-7-ethyl-2-benzofurnamethanol) (bufuralol);
(3-[3-acetyl-4-[tert.butylamino)-2-hydroxypropyl]phenyl]-1,1-diethylurea HCl) (celiprolol);
((±)-2-[2-[3-[(1,1-dimethylethyl)amino]-2-hydroxypropoxy]phenoxy]N-methylacetamide HCl) (cetamolol);
(2-benzimidazolyl-phenyl(2-isopropylaminopropanol));
((-)-3′-acetyl-4′-(2-hydroxy-3-isopropylaminopropoxy)acetanilide HCl) (diacetolol);
(methyl-4-[2-hydroxy-3-[(1-methylethyl)aminopropoxy]]benzenepropanoate HCl) (esmolol);
(erythro-DL-1-(7-methylindan-4-yloxy)-3-isopropylaminobutan-2-ol);
(1-(tert.butylamino)-3-[0-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2-hydroy-5-[(R)-1-hydroxy-2-[(R)-(1-methyl-3-phenylpropyl)amino]ethyl]benzamide);
(4-hydroxy-9-[2-hydroxy-3-(isopropylamino)-propoxy]-7-methyl-5H-furo[3,2-g][1]-benzopyran-5-one) (iprocrolol);
((-)-5-(tert.butylamino)-2-hydroxypropoxy]-3,4-dihydro1-(2H)-naphthalenone HCl) (levobunolol);
(4-(2-hydroxy-3-isopropylamino-propoxy)-1,2-benzisothiaole HCl);
(4-[3-(tert.butylamino)-2-hydroxypropoxy]-N-methylisocarbostyril HCl);
((±)-N-2-[4-(2-hydroxy-3-isopropyl aminopropoxy)phenyl]ethyl-N′-isopropyulurea) (pafenolol);
(3-[[(2-trifluoroacetamido)ethyl]amino]-1-phenoxypropan-2-ol);
(N-(3-(o-chlorophenoxy)-2-hydroxypropyl)-N′-(4′-chloro-2,3-dihydro-3-oxo-5-pyridazinyl)ethylenediamine);
((±)-N-[3-acetyl-4-[2-hydroxy-3-[1-methylethyl)amino]propoxy]phenyl]butanamide) (acebutolo);
((±)-4′-|3-(tert-butylamino)-2-hydroxypropoxy]spiro[cyclohexane-1,2′-indan]-1′-one) (spirendolol);
(7-[3-[[2-hydroxy-3-[-[(2methylindol-4-yl)oxy]propyl]amino]butyl]thiophylline) (teopropol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-(tert.butylamino)-2-hydroxy-propoxy)benzonitrile HCL) (bunitrolol);
((±)-2′-[3-(tert-butylamino)-2-hydroxypropoxy-5′-fluorobutyrophenone) (butofilolol);
(1-(carbazol-4-yloxy)-3-(isopropylamino)-2propanol) (carazolol);
(5-(3-tert.butylamino-2-hydroxy)propoxy-3,4k-dihydrocarbostyril HCl) (carteolol);
(1-(tert.butylamino)-3-(2,5-dichlorophenoxy)-2-propanol) (cloranolol);
(1-(inden-4(or 7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy-1-naphthyl)oxy]-2-propanol) (nadolol);
((S)-1-(2-cyclopentylphenoxy)-3-[(1,1-dimethylethy)amino]-2-propanol sulfate (2:1)) (penbutolol);
(4′-[1-hydroxy-2-(amino)ethyl]methanesulfonanilide) (sotalol);
(2-methyl-3-[4-(2-hydroxy-3-tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-B-methylcinnamonitrile) (pacrinolol);
((±)-2-(3′-tert.butylamino-2′-hydroxypropylthio)-4-(5′-carbamoyl-2′-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[3-(p-butoxyphenoxy)-2-hydroxypropyl]amino]ethyl]amino-1,3-dimethyluracil) (pirepolol);
(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);
(3,4-dihydro-8-(2-hydroxy-3-isopropylaminopropoxy)-3-nitroxy-2H-1-benzopyran) (nipradolol);

α and β-Adrenergic Blocking Agents:
((±)-1t-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol HCl);
(4-hydroxy-a-[[3-(4-methoxyphenyl)-1-methylpropyl]aminomethyl]-3-(methylsulfinyl)-benzmethanol HCl) (sulfinalol);
(5-[1-hydroxy-2-[[2-(o-methoxyphenoxy)ethyl]amino]ethyl]-2-methylbenzenesulfonamide HCl);

(5-[1-hydroxy-2-[1-methyl-3-phenylpropyl)amino]ethyl]salicylamide HCl) (labaetalol);
(1-((3-chloro-2-methyl-1H-indol-4-yl)oxy)-3-((2 phenoxyethyl)amino)-2-propanol-hydrogenmalonate) (ifendolol);
(4-(2-hydroxy-3-[(1-methyl-3-phenylpropyl)amino]propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)-3,4-dihydroxy)quinoxolin-2(1H)-one);

CNS-Acting Agents:
clonidine; methyldopa;

Adrenergic Neuron Blocking Agents:
guanethidine; reserpine and other rauwolfia alkaloids such as rescinnamine;

Vasodilators:
diazoxide; hydralazine; minoxidil;

Angiotensin I Converting Enzyme Inhibitors:
1-(3-mercapto-2-methyl-1-oxopropyl)-L-proline (captopril);
(1-(4-ethoxycarbonyl-2,4(R,R)-dimethylbutanoyl)indoline-2(S)-carboxylic acid);
(2-[2-[[1(ethoxycarbonyl)-3-phenyl-propyl]amino]-1-oxopropyl]-1,2,3,4-tetrahydro-3-isoquinoline carboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)cis,syn-octahydroindol-2(S)-carboxylic acid HCl)-((-)-(S)-1-[(S)-3-mercapto-2-methyl-1-oxopropy]indoline-2-carboxylic acid);
([1(S),4S]-1-[3-(benzoylthio)-2-methyl-1-oxopropyl]-4-phenylthio-L-proline
(3-([1-ethoxycarbonyl-3-phenyl-(1S)-propyl]amino)2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1(S)-carboxy-3-phenylpropyl]-L-lysyl-L-proline (lysinopril);
2-[N-[(S)-1-ethoxycarbonyl-3-phenylpropyl]-L-alanyl(1S,3S,5S)-2-azabicyclo[3.3.0]octane-3-carboxylic acid;
[1(S),4S]-1-(3-mercapto-2-methyl-1-oxopropyl)-4-phenylthio-L-proline S-benzoyl calcium salt (zofenopril);
[1-(+/-)4S]-4-cyclohexyl-1-[[2-methyl-1-[1-(1-(oxypropoxy)propoxy](4-phenylbutyl)phosphinyl]acetyl]L-proline sodium salt (fosfopril).

Calcium Channel Blockers:
nifedepine; nitrendipine, verapamil; diltiazam.

Other Antihypertensive Agents:
aminophylline; cryptenamine acetates and tannates; deserpidine; meremethoxylline procaine; pargyline; trimethaphan camsylate; and the like, as well as admixtures and combinations thereof.

Typically, the individual daily dosages for these combinations can range from about one-fifth of the minimally-recommended clinical dosages to the maximum recommended levels for the entities when they are given singly. Coadministration is most readily accomplished by combining the active ingredients into a suitable unit dosage form containing the proper dosages of each. Other methods of coadministration are, of course, possible.

The following Examples are intended to be representative and not limiting.

EXAMPLE 1

Preparation of BOC-N-(3-phenylpropyl)His-ACHPA-NH-(2(S)-methylbutane)

a. Boc-$N^{\alpha}$-(3-phenylpropyl)-$N^{\pi}$-BOM-histidine

A solution of $N^{\alpha}$-BOC-$N^{\pi}$-BOM-histidine in anhydrous TFA was allowed to stand for two hours at room temperature, with the residue after evaporation being reconcentrated twice from $CH_2Cl_2$ and then taken up in EtOH. To the solution were added 3-phenylpropionaldehyde (1.1 equiv) and $NaCNBH_3$ (1 equiv), and the mixture was stirred for 18 hours. The excess $NaCNBH_3$ was neutralized by the addition of 1N HCl, to bring the pH to 3, then the pH was readjusted to 10 by the addition of 1N NaOH. $BOC_2O$ (1 equiv) was added, and after 4 hours, the pH was adjusted to 5 by the addition of 1N HCl, and the solution was extracted several times with EtOAc. The combined organic portions were dried ($Na_2SO_4$), filtered, and evaporated to dryness, with the crude product being purified by chromatography on silica gel.

b. BOC-ACHPA-OH

BOC-ACHPA-OEt (4 g) was dissolved in methanol (15 mL) and $H_2O$ (5 mL) and the pH was adjusted to 10 with 1:1 pH 10 buffer:$CH_3OH$. 2N NaOH (2.1 equiv) was and added the mixture was allowed to stir for 2 hours, before the pH was adjusted to 10 by addition of 1N HCl, and the solution extracted with EtOAc. The solution was then cooled in ice, with EtOAc being added, and the aqueous layer was acidified to pH 2.5 with 6N HCl. The organic layer and several EtOAc extracts of the aqueous layer were combined, dried ($Na_2SO_4$) and evaporated to 3.5 g (93%) of a white brittle foam. TLC (silica gel; 80:10:1:1: $CHCl_3$: $CH_3OH$: $H_2O$:HOAc) showed a single spot, $R_f = 0.45$.

c. BOC-ACHPA-NH-(2(S)-methylbutyl)

To a cooled (ice-bath) solution of BOC-ACHPA-OH (3.5 g) in DMF (25 mL) was added $Et_3N$ (1.6 mL) and DPPA (2.5 ml), and the resulting mixture was stirred under nitrogen for 30 minutes. 2(S)-Methylbutylamine (1.36 ml) was added, and the reaction mixture was stirred and allowed to warm to room temperature. After 18 hours, the mixture was concentrated in vacuo and the residue taken up in EtOAc (250 mL). The solution was washed with 1N HCl, $H_2O$, saturated $NaHCO_3$, $H_2O$ and brine and then dried ($Na_2SO_4$), with the residue after evaporation being purified by chromatography on silica gel (6:4 hexanes:EtOAc), to give the product (3.75 g; 89%) as a colorless oil.

d. BOC-$N^\alpha$-(3-phenylpropyl)-$N^\pi$-BOM-His-ACHPA-NH-(2(S)-methylbutane)

The product of Step 1c was dissolved in anhydrous TFA and the solution allowed to stand at room temperature for 2 hours, after which the residue was reconcentrated twice from $CH_2Cl_2$. The crude product was combined with the product of Step 1a (1 equiv.), N-methylmorpholine (2 equiv), DCC (1.1 equiv) and HOBt (1 equiv) in DMF and the resulting solution was allowed to stir for 18 hours. The residue after evaporation was taken up in EtOAc and the solution extracted with aqueous $NaHCO_3$, $H_2O$, saturated aqueous citric acid, $H_2O$, and brine, then dried ($Na_2SO_4$), filtered and evaporated. The product was purified by chromatography on silica gel.

e. BOC-$N^\alpha$-(3-phenylpropyl)His-ACHPA-NH-[2(S)-methyl)butyl]

The product from Step 1d is taken up in EtOH and hydrogenated with Pd(C) (5% by wt) at 40 psi for 4 hours. The resulting product is purified by chromatography on silica gel.

EXAMPLE 2

Preparation of BOC-Phe-(S-benzyl)Cys-ACHPA-NH-[2(S)-methyl)butyl]

a. BOC-(S-benzyl)cysteine

This compound was prepared as described by Arnold, J. Amer. Chem Soc., 1985, 107, 7105-7109, from N-BOC-L-serine β-lactone and benzylmercaptan.

b. BOC-(S-benzyl)Cys-ACHPA-NH-[2(S)-methyl)butyl]

The product of Step 1c was treated with anhydrous TFA as described in Step 1d, then combined with BOC-(S-benzyl)cysteine (1 equiv), N-methylmorpholine (2 equiv), DCC (1 equiv) and HOBt (1 equiv) in DMF. The resulting mixture was stirred for 18 hours, then evaporated to dryness, with the residue being taken up in EtOAc. The solution was filtered, washed with aqueous $NaHCO_3$, $H_2O$, 0.5 N HCl, $H_2O$ and brine, then dried ($NaSO_4$), filtered and evaporated, and the product was purified by chromatography on silica gel.

c. BOC-Phe-(S-benzyl)Cys-ACHPA-NH-[2(S)-methyl)butyl]

A solution of the product of Step 2b in anhydrous TFA was allowed to stand at room temperature for 2 hours, with the residue after evaporation being reconcentrated twice from $CH_2Cl_2$, then combined with BOC-Phe-N-hydroxysuccinimide ester (1.1 equiv) and $Et_3N$ (2 equiv) in DMF. The solution was stirred for 6 hours and then evaporated to dryness, with the residue being chromatographed on silica gel, to give the product.

EXAMPLE 3

Preparation of BOC-Phe-(S-4-pyridyl)Cys-ACHPA-NH-(2(S)-methylbutyl)

BOC-Phe-(S-4-pyridyl)Cys-ACHPA-NH-(2(S)-methyl-butyl) is prepared according to the method of Example 2, with 4-mercaptopyridine replacing benzylmercaptan in Step 2(a).

EXAMPLE 4

Preparation of BOC-Phe-(S-1-methyltetrazol-5-yl)Cys-ACHPA-NH-(2(S)-methylbutyl)

BOC-Phe-(S-1-methyltetrazol-5-yl)Cys-ACHPA-NH-(2(S)-methylbutyl) is prepared according to the method of Example 2, with 1-methyl-5-mercaptotetrazole replacing benzylmercaptan in Step 2(a).

EXAMPLE 5

Preparation of BOC-Phe-(S-2,3-dihydroxypropyl)Cys-ACHPA-NH (2(S)-methylbutyl)

BOC-Phe-(S-2,3-dihydrocypropyl)Cys-ACHPA-NH-(2(S)-methylbutyl) is prepared according to the method of Example 2, with 3-mercapto-1,2-propanediol replacing benzylmercaptan in Step 2(a).

EXAMPLE 6

Preparation of BOC-Phe-(S-benzyl)Cys-ACHPA-Ile-NH-4-pyridylmethyl

BOC-Phe-(S-benzyl)Cys-ACHPA-Ile-NH-4-pyridylmethyl is prepared by the procedures outlined in example 2 and substituting Ile-4-aminomethylpyridine amide for 2(S)-methylbutylamine.

EXAMPLE 7

Preparation of BOC-Phe-(S-1-methyltetrazol-5-yl)Cys-ACHPA-Lys-NH-i-butyl

BOC-Phe-(S-1-methyltetrazol-5-yl)Cys-ACHPA-Lys-NH-i-butyl is prepared the procedures outlinded in example 4 and substituting Lys-i-butyl amide for 2(S)-methylbutylamine.

EXAMPLE 8

Preparation of BOC-Phe-(S-4-pyridyl)Cys-ACHPA-Ile-NH-(2(S)-methylbutyl)

BOC-Phe-(S-4-pyridyl)Cys-ACHPA-Ile-NH-(2(S)-methylbutyl) is prepared the procedures outlined in example 3 and substituting Ile-2(S)-methylbutyl amide for 2(S)-methylbutylamine.

**Claims**

1. A peptide of the formula:

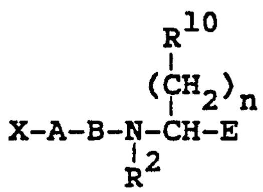

(I),

wherein

X is hydrogen, $C_1$-$C_5$-alkyl, $R^1OCH_2CO$, $R^1CH_2OCO$, $R^1OCO$, $R^1(CH_2)_nCO$, $(R^2)_2N(CH_2)_nCO$, $R^1SO_2(CH_2)_mCO$, $R^1SO_2(CH_2)_mOCO$, $R^1OCHCO$, $(R^1)_2CHCO$, $R^1NHCHCO$, or $R^1SCHCO$, where

$R^1$ is $C_1$-$C_6$-alkyl; $C_3$-$C_7$-cycloalkyl; aryl, wherein aryl is unsubstituted or mono-, di- or trisubstituted phenyl or naphthyl, where the substituent(s) is/are independently selected from the group consisting of amino mono- or di-$C_1$-$C_4$-alkylamino, amino-$C_1$-$C_4$-alkyl, hydroxy-$C_1$-$C_4$-alkyl, di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO,$CO_2H$,$CO_2$-$C_1$-$C_4$-alkyl, $NR^5R^6$, and $N(R^5)_3^{\oplus} A^{\ominus}$ wherein $R^5$ and $R^6$ are independently hydrogen, unsubstituted or monosubstituted $C_1$-$C_4$-alkyl, where the substituent is amino, $C_1$-$C_4$-alkylamino, di-$C_1$-$C_4$-alkylamino, hydroxyl, $C_1$-$C_4$-alkoxy or $N(C_1\text{-}C_4\text{-alkyl})_3^{\oplus} A^{\ominus}$, and $A^{\ominus}$ is chloride, bromide, hydroxide or acetate; Het, wherein Het is an unsubstituted or mono- or disubstituted 5- or 6-membered heterocyclic ring or benzofused 5- or 6- membered heterocyclic ring, where the one or two heteroatoms are independently selected from the group consisting of N, O, S, NO, SO, $SO_2$ or quarternized N, and the substituent(s) is/are independently selected from the group consisting of hydroxyl, thiol, $C_1$-$C_6$-alkyl, $CF_3$, $C_1$-$C_4$-alkoxy, 0alo, aryl, aryl-$C_1$-$C_4$-alkyl, amino, mono- or di-$C_1$-$C_4$-alkylamino, amino-$C_1$-$C_4$-alkylamino, amino-$C_1$-$C_4$-alkyl, hydroxy-$C_1$-$C_4$-alkyl, di-$C_1$-$C_4$-alkyl-amino-$C_1$-$C_4$-alkyl, guanidyl, guanidyl-$C_1$-$C_4$-alkyl, CHO, $CO_2H$, $CO_2$-$C_1$-$C_4$-alkyl, $NR^5R^6$, and $N(R^5)_3^{\oplus} A^{\ominus}$, wherein $R^5$,$R^6$ and $A^{\ominus}$ are as defined above, or 5-oxo-2-pyrrolidinyl;

$R^2$ is hydrogen or $C_1$-$C_5$-alkyl;

m is 1 to 5;

n is 0 to 5;

A is absent;

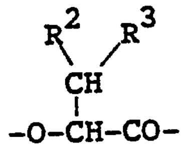

where

$R^3$ is:

hydrogen; $C_1$-$C_5$-alkyl; $C_3$-$C_7$-cycloalkyl; 1- or 2-adamantyl; $(CH_2)_p$-aryl, wherein aryl is as defined above and p is 1 to 3; or $(CH_2)_p$-Het, wherein Het and p are as defined above; and

$R^2$ is as defined above;

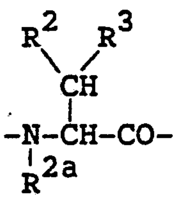

where

$R^{2a}$ is hydrogen or $C_1$-$C_5$-alkyl; and

$R^2$ and $R^3$ are as defined above;

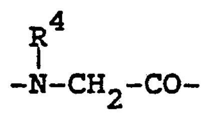

where

$R^4$ is hydrogen, $C_1$-$C_5$-alkyl or aryl-$C_1$-$C_5$-alkyl, wherein aryl is as defined above;

B is:

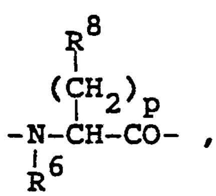

where

$R^6$ is aryl-substituted $C_1$-$C_5$-alkyl, wherein aryl is as defined above, $C_5$-$C_7$ cycloalkyl, or Het, as defined above;

$R^8$ is unsubstituted or monosubstituted $C_1$-$C_4$-alkyl,
wherein the substituent is carboxyl, amino, hydroxyl, guanidino, $C_1$-$C_4$-alkoxycarbonyl, Het, as defined above, or aryl, as defined above; and

p is as defined above;

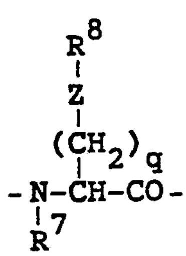

where

$R^7$ is hydrogen, unsubstituted or aryl-substituted $C_1$-$C_5$-alkyl, wherein aryl is as defined above, $C_5$-$C_7$-cycloalkyl, or Het, as defined above;

Z is O, S, $NR^2$, $NR^2$-CO,CO-$NR^2$, SO, $SO_2$, wherein $R^2$ is as defined above;

q is 0 to 3; and

$R^8$ is as defined above;

except that $-(CH_2)_{q'}$-Z-$R^8$ cannot be $-CH_2$-$SCH_3$;

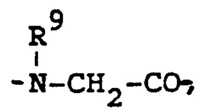

where

$R^9$ is $C_1$-$C_4$-alkyl, substituted by $R^8$ or Z-$R^8$, wherein $R^8$ and Z are as defined above;

except that $R^9$ cannot be hydrogen, $C_1$-$C_5$-alkyl or aryl-$C_1$-$C_5$-alkyl;

$R^{10}$ is $-CHR^{11}R^{11a}$;

where

$R^{11}$ and $R^{11a}$ are related such that when $R^{11}$ is hydrogen, $C_1$-$C_5$-alkyl, aryl, as defined above, Het, as defined above, unsubstituted or mono-, di or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy, and halo; $C_1$-$C_3$-alkoxy, or $C_1$-$C_3$-alkylthio, then $R^{11a}$ is hydrogen,

or when $R^{11}$ is hydroxy-$C_1$-$C_4$-alkyl, amino-$C_1$-$C_4$-alkyl, or $C_1$-$C_4$-alkyl, then $R^{11a}$ is hydrogen or $C_1$-$C_3$-alkyl;

W<br>
E is - CH-G,

where

W is OH, $NH_2$ or $OR^{12}$, wherein $R^{12}$ is $C_1$-$C_4$-alkanoyl or $C_1$-$C_6$-alkanoyloxy-$C_1$-$C_4$-alkyl; and

G is Q-CO-T-U-V,

wherein

Q is a single bond; CH-$R^{13}$, where $R^{13}$ is hydrogen; aryl, as defined above: $C_1$-$C_8$-alkyl; $C_3$-$C_7$-cycloalkyl; mono- or disubstituted $C_2$-$C_8$-alkyl, where the substituent(s) is/are independently selected from the group consisting of hydroxy; $CO_2H$; $CO_2$-$C_1$-$C_4$-alkyl; $CONR^5R^6$, wherein $R^5$ and $R^6$ are as defined above; amino; mono- or di-$C_1$-$C_4$-alkylamino; and guanidyl, and the substitution occurs on the last 1 or 2 carbon atoms of the alkyl chain; or mono- or disubstituted aryl or $C_3$-$C_7$-cycloalkyl, where the substituent(s) is/are independ ently selected from the group consisting of $C_1$-$C_4$-alkyl, trifluoromethyl, hydroxy, $C_1$-$C_4$-alkoxy, and halo; or $CH_2CH$-$R^{13a}$, where $R^{13a}$ is $CHR^{14}R^{14a}$, wherein $R^{14}$ is hydrogen; amino; hydroxyl; $C_1$-$C_5$-alkyl; $C_3$-$C_7$-cycloalkyl; aryl, as defined above; aryl-$C_1$-$C_4$-alkyl; Het, as defined above; Het-$C_1$-$C_4$-alkyl; amino-$C_1$-$C_4$-alkyl; or guanidyl-$C_1$-$C_4$-alkyl; and $R^{14a}$ is hydrogen or $C_1$-$C_5$-alkyl;

T and U are independently absent or

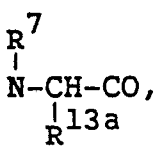

where $R^7$ and $R^{13a}$ are as defined above; and

V is O-$(CH_2)_uR^{16}$, where $R^{16}$ is hydrogen; aryl, as defined above; Het, as defined above; or unsubstituted or mono- or disubstituted $C_2$-$C_4$-alkyl, $C_3$-$C_7$-cycloalkyl or $C_2$-$C_7$-cycloalkyl-$C_1$-$C_4$-alkyl, wherein the substituent(s) is/are independently selected from the group consisting of amino, hydroxyl, $C_1$-$C_4$ alkoxy, thio, halo, mono- or di-$C_1$-$C_4$-alkylamino, $CO_2H$, or $CONR^5R^6$, where $R^5$ and $R^6$ are as defined above; and u is 0 to 5;

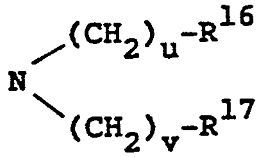 ;

where $R^{17}$ is hydrogen; aryl, as defined above; Het, as defined above; unsubstituted or mono- or disubstituted $C_2$-$C_4$-alkyl, $C_3$-$C_7$-cycloalkyl or $C_3$-$C_7$-cycloalkyl-$C_1$-$C_4$- alkyl, wherein the substituent(s) is/are independently selected from the group consisting of amino; hydroxyl; $C_1$-$C_4$-alkoxy; thio; halo; mono- or di-$C_1$-$C_4$-alkylamino; $CO_2H$; or $CONR^5R^6$, where $R^5$ and $R^6$ are as defined above;

v is 0 to 5; and

$R^{16}$ and u are as defined above; or

$R^{16}$ and $R^{17}$ are joined to form an unsubstituted or mono- or disubstituted 5- to 7-membered heterocyclic ring or benzofused 5- to 7-membered heterocyclic ring, which is either saturated or unsaturated, wherein the one to three heteroatoms is/are independently selected from the group consisting of nitrogen, oxygen and sulfur, and the substituent(s) is/are independently selected from the group consisting of $C_1$-$C_6$-alkyl; hydroxyl; trifluoromethyl; $C_1$-$C_4$-alkoxy; halo; aryl, as defined above; aryl-$C_1$-$C_4$-alkyl; amino; thio; and mono- or di-$C_1$-$C_4$-alkylamino;

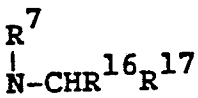 ,

where $R^7$, $R^{16}$ and $R^{17}$ are as defined above, or $R^{16}$ and $R^{17}$ are joined as defined above; or $O$-$CHR^{16}R^{17}$, where $R^{16}$ and $R^{17}$ are as defined above, or are joined as defined above; or

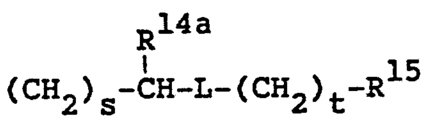 ,

wherein

$R^{15}$ is hydrogen; unsubstituted or monosubstituted $C_1$-$C_6$-alkyl, where the substituent is amino or hydroxyl; aryl-$C_1$-$C_4$-alkyl, where aryl is as defined above; $C_5$-$C_7$-cycloalkyl-$C_1$-$C_4$-alkyl; $C_1$-$C_5$-alkenyl; or Het, as defined above:

L is a single bond, S, SO, $SO_2$, $NR^7$, or $NR^7$-CO,

where $R^7$ is as defined above;

s is 0 to 2;

t is,0 to 2; and

$R^{14a}$ is as defined above;

$CH_2$-$NR^{12a}$-M,

where

$R^{12a}$ is hydrogen, $C_1$-$C_5$-alkyl, $SO_2$-phenyl, formyl, or $CO_2$-$C_1$-$C_4$-alkyl; and $R^{13a}$

M is CH-CO-T-U-V,

wherein

$R^{13a}$, T, U and V are as defined above;

or

$CO$-$CF_2$-$CO$-T-U-V

where

T, U and V are as defined above;

or a pharmaceutically-acceptable salt thereof.

2. A peptide according to Claim 1, wherein

E is 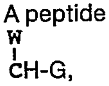,

where W is OH and G is Q-CO-T-U-V, wherein Q is $CH_2$ or

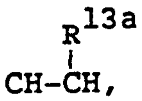

where $R^{13a}$ is isopropyl or isobutyl, and either T or U is

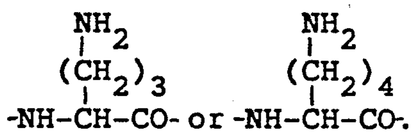

3. A peptide according to Claim 1, selected from the group consisting essentially of:
BOC-(4-methoxy)Phe-[S-(2-amino-1,3,4-thiadiazol-5-yl)]Cys-ACHPA-NH-(2(S)-methylbutyl);
BOC-Phe-[S-(1,2,4-triazol-3-yl)]cys-AmACHPA-NH-(2(S)-methylbutyl);
BOC-Phe-[S-2-aminothiazol-4-Yl]Cys-ACHPA-AMP;
BOC-N-(3-phenylpropyl)His-(2-allyl)ACHPA-NH-(2(S)-methylbutyl);
BOC-Phe-[S-(1,2,4-triazol-3-yl]Cys-ACHPA-NH-([2(S)-methylbutyl);
BOC-Phe-[S-(4-pyridyl)]Cys-ACHPA-NH-[2(s)-methylbutyl];
BOC-Phe-Glu(qN,N-diethylamide]-ACHPA-NH-(2(S)-methylbutyl);
CBZ-Phe-[S-(3-amino-1,2,4-triazol-5-yl)]Cys-Cal[CH(OH)$CH_2$]Val-Leu-AMP;
BOC-Phe-[S-(1-methyltetrazol-5-yl)]Cys-ACHPA-NH-(2(S)-methylbutyl);
BOC-Phe-[3-(N-benzylpyridin-4-yl)amino]Ala-ACHPA-Ile-AMP;
BOC-Phe-[S-(4-phenylthiazol-2-yl)]Cys-Cal[$CH_2$NH] Val-AMP;
BOC-[3-(1-Naphthyl)]Ala-S-(imidazol-2-yl)]Cys-ACHPA-NH-(2(S)-methylbutyl);
BOC-Phe-[S-(1,2,4-triazol-3-yl)]Cys-ACHPA-Lys-NH-i-butyl;
BOC-Phe-[S-(3-amino-1,2,4-triazol-5-yl)]Cys-ACHPA-Lys-NH-(2(S)-methylbutyl);
BOC-(p-O-methyl)Phe-[S-(1,2,4-triazol-3-yl)]Cys-Cal[CH (OH)$CH_2$]Val-Lys-NH-i-butyl;
BOC-Phe-[S-(1,2,4-triazol-3-yl)]Cys-Cal[CH(OH)$CH_2$]Val-Lys-O-t-butyl;
BOC-Phe-[S-(1,2,4-triazol-3-yl)]Cys-ACHPA-Ile-Lys-NH-i-butyl;
BOC-Phe-[S-(2-aminothiazol-3-yl)]Cys-ACHPA-Lys-NH-i-butyl;
BOC-Phe-[S-(1,2,4-triazol-3-yl)]Cys-ACHPA-Lys-Pro-$OCH_3$;
BOC-Phe-[S-(1,2,4-triazol-3-yl)]Cys-ACHPA-Lys-Phe-OH;
BOC-Phe-[S-(1,2,4-triazol-3-yl)]Cys-ACHPA-Lys-NH-i-butyl;
BOC-Phe-[S-(2-aminothiazol-3-yl)]Cys-ACHPA-Ile-Lys-;
BOC-Phe-[S-(2-aminothiazol-3-yl)]Cys-ACHPA-Lys-Lys-NH-i-butyl;
BOC-Phe-[S-(2-aminothiazol-3-yl)]Cys-ACHPA-Lys-NH-((1-hydroxymethyl)-5-aminopentyl];
BOC-Phe-Glu[q2-N,N-diethylamide]-ACHPA-Lys-NH-i-butyl;
BOC-Phe-Lys[Ne-acetyl]-ACHPA-Lys-NH-i-butyl;
BOC-Phe-Orn[Ne-acetyl]-ACHPA-Lys-NH-i-butyl; and
BOC-Phe-Lys[Ne-benzoyl]-ACHPA-Lys-NH-i-butyl.

4. A Composition comprising a therapeutically-effective amount of a peptide according to Claim 1, and a pharmaceutical carrier.

5. A composition according to Claim 4, also comprising one or more antihypertensive agents selected from the group consisting essentially of:

Diuretics:
acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents:
dibenamine; phentolamine; phenoxybenzamine; prazosin; tolazoline;

β-Adrenergic Blocking Agents:
atenolol; metoprolol; nadolol; propranolol; timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);

(((±)-1-(4-((2-isopropoxyethoxy)methyl)phenoxy)-3-iso-propylamino-2-propanol)fumarate) (bisoprolol); (4-(2-hydroxy-3-[4-(phenoxymethyl)-piperidino]propoxy)-indole); (carbazolyl-4-oxy-5,2-(2-methoxyphenoxy)-ethylamino-2-propanol); (1-((1,1-dimethylethyl)amino)-3-((2-methyl-1H-indol-4-yl)oxy)-2-propanol benzoate) (bopindolol); (1-(2-exobicyclo[2.2.1]-hept-2-ylphenoxy)-3-[(1-methylethyl)-amino]-2-propanol HCl) (bornaprolol); (o-[2-hydroxy-3-[(2-indol-3-yl-1,1-dimethylethyl)amino]propoxy]benzonitrile HCl) (bucindolol); (a-[(tert.butylamino)methyl]-7-ethyl-2-benzofuranmethanol) (bufuralol); (3-[3-acetyl-4-[3-(tert.butylamino)-2-hydroxypropyl]phenyl]-1,1-diethylurea HCl) (celiprolol); ((±)-2-[2-[3-[(1,1-dimethylethyl)amino]-2-hydroxypropoxy]phenoxy]-N-methylacetamide HCl) (cetamolol); (2-benzimidazolyl-phenyl(2-isopropylaminopropanol)); ((±)-3′-acetyl-4′-(2-hydroxy-3-isopropylaminopropoxy)(methyl-4-[2-hydroxy-3-[(1-methylethyl)amino-propoxy]]benzenepropanoate HCl) (esmolol); (erythro-DL-1-(7-methylindan-4-yloxy)-3-isopropylaminobutan-2-ol); (1-(tert.butylamino)-3-[O-(2-propynyloxy)phenoxy]-2-propanol (pargolol); (1-(tert.butylamino)-3-[o-(6-hydrazino-3-pyridazinyl)phenoxy]-2-propanol diHCl) (prizidilol); ((-)-2-hydroxy-5-[(R)-1-hydroxy-2-[(R)-(1-methyl-3-phenylpropyl)amino]ethyl]benzamide); (4-hydroxy-9-[2-hydroxy-3-(isopropylamino)-propoxy]-7-methyl-5H-furo[3,2-g][1]-benzopyran-5-one) (iprocrolol); ((-)-5-(tert.butylamino)-2-hydroxypropoxy]-3,4-dihydro1-(2H)-naphthalenone HCl) (levobunolol); (4-(2-hydroxy-3-isopropylamino-propoxy)-1,2-benzisothiazole HCl); (4-[3-(tert.butylamino)-2-hydroxypropoxy]-N-methylisocarbostyril HCl); ((±)-N-2-[4-(2-hydroxy-3-isopropyl aminopropoxy)phenyl]ethyl-N′-isopropylurea) (pafenolol); (3-[[(2-trifluoroacetamido)ethyl]amino]-1-phenoxypropan-2-ol); (N-(3-(o-chlorophenoxy)-2-hydroxypropyl)-N′-(4′-chloro-2,3-dihydro-3-oxo-5-pyridazinyl)ethylenediamine); ((±)-N-[3-acetyl-4-[2-hydroxy-3-[(1-methylethyl)amino]propoxy]phenyl]butanamide) (acebutolol): ((±)-4′-[3-(tert-butylamino)-2-hydroxypropoxy]spiro[cyclohexane-1,2′-indan]-1′-one) (spirendolol); (7-[3-[[2-hydroxy-3-[(2-methylindol-4-yl)oxy]propyl]amino]butyl]thiophylline) (teoprolol); ((±)-1-tert.butylamino-3-(thiochroman-8-yloxy)-2-propanol) (tertatolol); ((±)-1-tert.butylamino-3-(2,3-xylyloxy)-2-propanol HCl) (xibenolol); (8-[3-(tert.butylamino)-2-hydroxypropoxy]-5-methylcoumarin) (bucumolol); (2-(3-(tert.butylamino)-2-hydroxy-propoxy)benzonitrile HCl) (bunitrolol); ((±)-2′-[3-(tert-butylamino)-2-hydroxypropoxy-5′-fluorobutyrophenone) (butofilolol); (1-(carbazol-4-yloxy)-3-(isopropylamino)-2-propanol) (carazolol); (5-(3-tert.butylamino-2-hydroxy)propoxy-3,4-dihydrocarbostyril HCl) (carteolol); (1-(tert.butylamino)-3-(2,5-dichlorophenoxy)-2-propanol) (cloranolol); (1-(inden-4(or 7)-yloxy)-3- isopropylamino)-2-propanol HCl) (indenolol); (1-isopropylamino-3-[(2-methylindol-4-yl)oxy]-2-propanol) (mepindolol); (1-(4-acetoxy-2,3,5-trimethylphenoxy)-3-isopropylaminopropan-2-ol) (metipranolol); (1-(isopropylamino)-3-(o-methoxyphenoxy)-3-[(1-methylethyl)amino]-2-propanol) (moprolol); ((1-tert.butylamino)-3-[(5,6,7,8-tetrahydro-cis-6,7-dihydroxy-1-naphthyl)oxy]-2-propanol) (nadolol); ((S)-1-(2-cyclopentylphenoxy)-3-[(1,1-dimethylethyl)amino]-2-propanol sulfate (2:1)) (penbutolol); (4′-[1-hydroxy-2-(amino)ethyl]methanesulfonanilide) (sotalol); (2-methyl-3-[4-(2-hydroxy-3-tert.butylaminopropoxy)phenyl]-7-methoxy-isoquinolin-1-(2H)-one); (1-(4-(2-(4-fluorophenyloxy)ethoxy)phenoxy)-3-iso-propylamino-2-propanol HCl); ((-)-p-[3-[(3,4-dimethoxyphenethyl)amino]-2-hydroxypropoxy]-β-methylcinnamonitrile) (pacrinolol); ((±)-2-(3′-tert.butylamino-2′-hydroxypropylthio)-4-(5′-carbamoyl-2′-thienyl)thiazole HCl) (arotinolol); ((±)-1-[p-|2-(cyclopropylmethoxy)ethoxy]phenoxy]-3-(isopropylamino)-2-propanol) (cicloprolol); ((±)-1-[(3-chloro-2-methylindol-4-yl)oxy]-3-[(2-phenoxyethyl)amino]-2-propanol) (indopanolol); ((±)-6-[[2-[[3-(p-butoxyphenoxy)-2-hydroxypropyl]amino]ethyl]amino]-1,3-dimethyluracil) (pirepolol); (4-(cyclohexylamino)-1-(1-naphtholenyloxy)-2-butanol); (1-phenyl-3-[2-[3-(2-cyanophenoxy)-2-hydroxypropyl]aminoethyl]hydantoin HCl); (3,4-dihydro-8-(2-hydroxy-3-isopropylaminopropoxy)-3-nitroxy-2H-1-benzopyran) (nipradolol);

α and β-Adrenergic Blocking Agents:

((±)-1-tert-butylamino)-3-[o-[2-(3-methyl-5-iso-xazolyl)vinyl]phenoxy]-2-propanol) (isoxaprolol); (1-isopropylamino-3-(4-(2-nitroxyethoxy)phenoxy)-2-propanol HCl); (4-hydroxy-α-[[3-(4-methoxyphenyl)-1-methylpropyl]aminomethyl]-3-(methylsulfinyl)-benzmethanol HCl) (sulfinalol); (5-[1-hydroxy-2-[[2-(o-methoxyphenoxy)ethyl]amino]ethyl]-2-methylbenzenesulfonamide HCl); (5-[1-hydroxy-2-[(1-methyl-3-phenylpropyl)amino]ethyl]salicylamide HCl) (labetalol); (1-((3-chloro-2-methyl-1H-indol-4-yl)oxy)-3-((2-phenoxyethyl)amino)-2-propanol-hydrogenmalonate) (ifendolol); (4-(2-hydroxy-3-[(1-methyl-3-phenylpropyl)amino]propoxy)benzeneacetamide);

(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)3,4-dihydroxy)quinoxolin-2(1H)-one);

CNS-Acting Agents:
clonidine; methyldopa;

Adrenergic Neuron Blocking Agents:
guanethidine; reserpine and other rauwolfia alkaloids such as rescinnamine;

Vasodilators:
diazoxide; hydralazine; minoxidil;

Angiotensin I Converting Enzyme Inhibitors:
1-(3-mercapto-2-methyl-1-oxopropyl)-L-proline (captopril);
(1-(4-ethoxycarbonyl-2,4(R,R)-dimethylbutanoyl)indoline-2(S)-carboxylic acid);
(2-[2-[[1-(ethoxycarbonyl)-3-phenyl-propyl]amino]-1-oxopropyl]-1,2,3,4-tetrahydro-3-isoquinoline carboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1-(S)-carboxy-3-phenylpropyl]-L-lysyl-L-proline (lysinopril);
2-[N-[(S)-1-ethoxycarbonyl-3-phenylpropyl]-L-alanyl(1S,3S,5S)-2-azabicyclo[3.3.0]octane-3-carboxylic acid;
[1(S),4S]-1-(3-mercapto-2-methyl-1-oxopropyl)-4-phenylthio-L-proline S-benzoyl calcium salt (zofenopril);
[1-(+/-)4S]-4-cyclohexyl-1-[[2-methyl-1-[1-(propoxy)propoxy](4-phenylbutyl)phosphinyl]acetyl]L-proline sodium salt (fosfopril);

Calcium Channel Blockers:
nifedepine; nitrendipine, verapamil; diltiazam;

Other Antihypertensive Agents:
aminophylline; cryptenamine acetates and tannates; deserpidine; meremethoxylline procaine; pargyline; trimethaphan camsylate;
and the like, as well as admixtures and combinations thereof.

6. The use of a peptide according to Claim 1 for the preparation of a medicament useful for treating renin-associated hypertension or renin-associated congestive heart failure.